# EUROPEAN PATENT APPLICATION

(11) **EP 1 116 476 A2**
(43) Date of publication of application: **18.07.2001**
(21) Application number: 01201517.8
(22) Date of filing: 24.03.1992
(51) Int. Cl.: A61B 18/22

(54) **Apparatus using a laser lucent needle**

(30) Priority: 05.04.1991 US 681225
(62) Divisional of application: 97104255.1
(71) Applicant: INDIGO MEDICAL, INCORPORATED, Palo Alto, CA 94303 (US)
(72) Inventor: Conn, Richard L., Albuquerque, New Mexico 87107 (US); Loree, Thomas Robert, Santa Fe, New Mexico 87501 (US); Bigio, Irving Joseph, Los Alamos, New Mexico 87544 (US); Strobl, Karlheinz, Santa Fe, New Mexico 87501 (US)
(74) Representative: Mercer, Christopher Paul

(57) **Abstract**

The present invention relates to apparatus for laser-induced hyperthermis or photodynamic therepy, more particularly to an apparatus combining an optical fiber with a laser lucent needle.

As is well known, lasers produce an intense beam of electromagnetic radiation of high spectral purity which can be collimated to a fine degres with high radiation densities, small angular divergences, and long coherence length. Such properties make lasers particularly attractive for a variety of medical uses.

The use of lasers to induce hyperthermia in tissue involves the insertion of an optical fiber into tissue. Without any additional protection, the fiber must be able to withstand insertion and withdrawal forces without fracturing or shedding material. Any accident during hyperthermia which leaves behind material which may not be easily retrieved could be disastrous.

## Description

### BACKGROUND AND SUMMARY OF THE INVENTION

The present invention relates to apparatus for laser-induced hyperthermia or photodynamic therapy, more particularly to an apparatus combining an optical fiber with a laser lucent needle.

As is well known, lasers produce an intense beam of electromagnetic radiation of high spectral purity which can be collimated to a fine degree with high radiation densities, small angular divergences, and long coherence length. Such properties make lasers particularly attractive for a variety of medical uses.

The use of lasers to induce hyperthermia in tissue involves the insertion of an optical fiber into tissue. Without any additional protection, the fiber must be able to withstand insertion and withdrawal forces without fracturing or shedding material. Any accident during hyperthermia which leaves behind material which may not be easily retrieved could be disastrous.

Additionally, it is desirable to control the diffusion of laser energy transmitted from the end of the optical fiber. The amount of scattering of laser energy in a particular tissue can be a critical factor in hyperthermia treatment. The degree of tissue scattering will determine the required diffusion of the energy transmitting end of the optical fiber. Therefore, it is desirable to provide a means for controlling the energy transmitted by the end of the optical fiber by diffusing the radiated energy for reduced energy density at the surface of the needle. This provides a needle energy delivery system that will prevent the development and deposit of charred materials on its surface while providing a design that will resist fracturing and the shedding of material. The reduction in energy density achieved by these techniques allows control of the thermal profile, which not only prevents charring and blocking of the photons, but also allows the optimization of hyperthermia therapy required to achieve the desired clinical results.

Alternative uses of the laser technology include the use of lasers to treat tumors, where the requirement of matching the heated volume to the size of the tumor can best be met by an apparatus in which the irradiation wavelength, and thus the penetration depth of the laser energy, can be changed. Although fluorescent dyes are useful for the conversion of the irradiation wavelength, the introduction of generally carcinogenic dyes into human tissue can cause serious health risks. Therefore, it is desirable to provide an apparatus which utilizes fluorescent dyes to convert the irradiation wavelength, while preventing the dye from leaking into the surrounding tissue.

It is a feature of the present invention to space the needle shaft from the internally disposed optical fiber. This permits liquid or gas cooling of the fiber, the needle and the adjacent tissue and permits the introduction of scatterers, diffusers or wavelengthshifting dyes within the interior of the needle. The ability to cool the tissue adjacent to the inserted needle enables improved control over the thermal profile in the tissue volume, which can be critical in hyperthermia. Encasement of an optical fiber in a needle is itself known, for example, in U.S. Patent No. 4,336,809, issued to Clark, a transparent cladding is in close contact with an optical fiber core. The optical needle disclosed by Clark acts as a linear side radiator.

A probe for photoepilation is disclosed in U.S. Patent No. 3,834,391, issued to Block. In that patent, a long, shallow tapered body terminates in a tapered tip, which holds the energy transmitting end of the fiber. The polished end of the fiber is flush with the exterior surface of the body. The specification of the Block patent cautions that the user must be careful in handling the probe because breakage of the sheathed fiber is possible.

A laser optic device for the treatment of blood vessels is disclosed in U.S. Patent No. 4,564,011, issued to Goldman. An optical fiber is concentrically disposed within a catheter, upon which a tapered probe is mounted such that the optical fiber itself is not in a penetrating portion in use. Laser energy is directed by a lens through the needle to an area approximately the size of the diameter of the needle.

U.S. Patent No. 4,905,689, issued to Stack, et al., discloses means for directing a laser beam circumferentially through windows that are concentrically disposed around the conical tip of the needle, rather than through a substantial portion of the shaft of the needle itself. In a preferred embodiment, the windows are formed from sapphires, rubies or diamonds and allow the laser beam to be directed to atherosclerotic plaque.

Finally, U.S. Patent No. 3,467,098, issued to Ayres, discloses a hand stylus connected to a remote laser source by a closed conduit but which does not encase an optical fiber, nor is the device insertable like a needle.

The present invention provides a laser lucent needle which may be used as a sheath for the insertion of optical fibers and diffusing tips, and then retrieved completely intact without a significant risk of leaving foreign material behind in the tissue. The optical fiber is positioned within a laser transparent needle which permits the transmission of laser energy through the walls of the needle. Any optical fiber fragments resulting from the fracturing or shedding of the fiber are thereby minimized or eliminated. This eliminates a significant risk of leaving foreign material behind in the tissue. Consequently, the transparent needle permits the use of inexpensive and reusable optical fibers and diffusion tips.

Another distinct advantage of the present invention is that the configuration of the transparent needle may be adapted to control the diffusion of the energy transmitted from the optical fiber. Scatterers, such as small plastic balls loaded with refractive scattering powder, may be placed in the tip of the needle to diffuse the energy transmitted from the fiber. Alternatively, high-refractive-index powder or material may be positioned within the walls and the end of the needle to provide a means for diffusing the laser energy. These embodiments of the present invention not only lower the power density at the tissue interface to increase the safety of hyperthermia, but also simplify the design and manufacturing of the optical fiber.

Specifically, the present invention comprises an apparatus for the transmission of laser energy to tissue including a hollow, transparent needle having a shaft and a tip, and adapted to transmit laser energy at least through a substantial portion of the length of the shaft. At least one optical fiber is disposed within the needle and extends into a penetrating portion of the needle, the fiber being adapted to transmit laser energy from one of its ends. Means, such as a spacer can be disposed within the needle to retain the fiber in a particular position within the needle, for example centrally. The needle has a configuration such that its interior surface is spaced from the exterior surface of the fiber.

In a specific embodiment, one end of the needle is closed and tapered. Means for scattering the laser energy transmitted from the optical fiber may be positioned within the closed end of the needle to diffuse energy transmitted from the fiber. Alternatively, an energy converter may be placed in the same position within the needle to convert the wavelength of energy transmitted from the fiber and alter its penetration depth. The placement of either refractive scattering material or energy converting material positioned within the walls and the closed end of the needle itself are further alternative embodiments.

In another embodiment, the needle is open-ended. The needle can be fitted with a solid, removable stylus to facilitate insertion, after which the stylus can be withdrawn and replaced by either an optical fiber or by a specialized instrument for such uses as obtaining biopsies or spectrographic tissue analysis. Such a procedure can be conducted before or after hyperthermia treatment of the tissue by replacing optical fiber in the needle with such a specialized instrument or vice-versa.

These alternative embodiments of the transparent needle provide a means to customize the needle for differing hyperthermia and/or surgical or clinical requirements. However, the basic embodiment of the present invention consistently provides an inexpensive, disposable or reusable means to protect the optical fiber from insertion and withdrawal forces and to reduce the risk of introducing fiber fragments into the tissue. Ultimately, the transparent needle and laser lucent apparatus of the present invention make it feasible to use less expensive laser equipment in the clinical application of laser energy interstitially in living systems.

In still another embodiment of the present invention, means are provided for the hyperthermia treatment of benign prostate hypertrophy (BPH). While it is well known that lasers can be used to induce hyperthermia in tissue, almost all of the clinical applications for such hyperthermia have been associated with the treatment of malignant tumors. Diffusing tips have been designed by others and used for photodynamic therapy in volumes of fluids, such as inside the bladder. Current techniques for hyperthermia of the prostate involve microwaves and heated probes; diffusion of laser light during interstitial applications has not been standardized in practice. The present invention facilitates the treatment of BPH with laser-induced hyperthermia. In accordance with an embodiment of the present invention, an optical fiber with a diffusing tip is inserted into the lucent needle which has been inserted directly into the prostate through, for example, a rectal approach under ultrasonic visualization and guidance. Particularly in such an application, integrity of the components is critical. Portions of the tip must not be broken off and left embedded in the tissue. The fiber mount itself must be able to withstand insertion and withdrawal without fracturing or shredding material. An accident during hyperthermia that leaves material behind within the prostate could be disastrous since there is no easy way to retrieve this material.

Use of concepts of this invention, can assure proper and safe diffusion of the light within the prostate tissue. By inserting a transparent needle into the prostate (or tumor) as a sheath for the insertion of an optical fiber and diffusing tip, retrieval can be complete without any significant risk of leaving foreign material behind, and with minimum side effects.

### BRIEF DESCRIPTION OF THE DRAWINGS

These and other features of the present invention may be more fully understood from the following detailed description, taken together with the accompanying drawings, wherein similar referenced characters refer to similar elements throughout and in which:
FIGURE 1 is a partial sectional view of an apparatus according to a preferred embodiment of the present invention, illustrating the incorporation of the diffusing tip placed on the energy transmitting end of the optical fiber;
FIGURE 2 is a partial sectional view of an apparatus of this invention including a spherical scatterer positioned adjacent to the closed end of the transparent needle;
FIGURE 3 is a partial sectional view of an apparatus of this invention illustrating the incorporation of a reflector positioned adjacent to the closed end of the needle and a spacer positioning of the transmitting end of the optical fiber;
FIGURE 4 is a partial sectional view of an apparatus of this invention illustrating the incorporation of multiple optical fibers and a scatterer which is adapted to retain the energy transmitting ends of the fibers in position within the needle;
FIGURE 5 is a partial sectional view of an apparatus of this invention with refractive scattering material positioned within the walls and closed end of the needle;
FIGURE 6 is a partial sectional view of an apparatus of this invention illustrating the incorporation of a lens at the energy transmitting end of the optical fiber;
FIGURE 7 is a partial sectional view of a double needle apparatus of this invention including a liquid contained within the core of the interior needle;
FIGURE 8 is a partial sectional view of an apparatus of this invention illustrating the placement of an energy wavelength converter in the closed end of the needle;
FIGURE 9 is a partial sectional view of an apparatus of this invention incorporating energy converting material within the walls and the closed end of the needle;
FIGURE 10 is a partial sectional view of a simple, basic configuration where additional diffusion means are not required;
FIGURE 11 is a partial sectional view of a simple, basic, multiple-fiber configuration;
FIGURE 12 is a partial sectional view of an open-ended configuration, containing a removable stylus which can be used during insertion; and
FIGURE 13 is a schematic view of the use of apparatus of this invention in the hyperthermia treatment of BPH.

### DESCRIPTION OF THE ILLUSTRATIVE EMBODIMENT

A detailed illustrative embodiment of the present invention is disclosed herein; however, it should be recognized that various structural elements may be embodied in a wide variety of forms, some of which may be quite different from those specific structural and functional details disclosed herein. Consequently, the details disclosed herein are merely representative; yet, in that regard, they are deemed to afford the best embodiment for the purposes of disclosure to provide a basis for the claims herein which define the scope of the present invention.

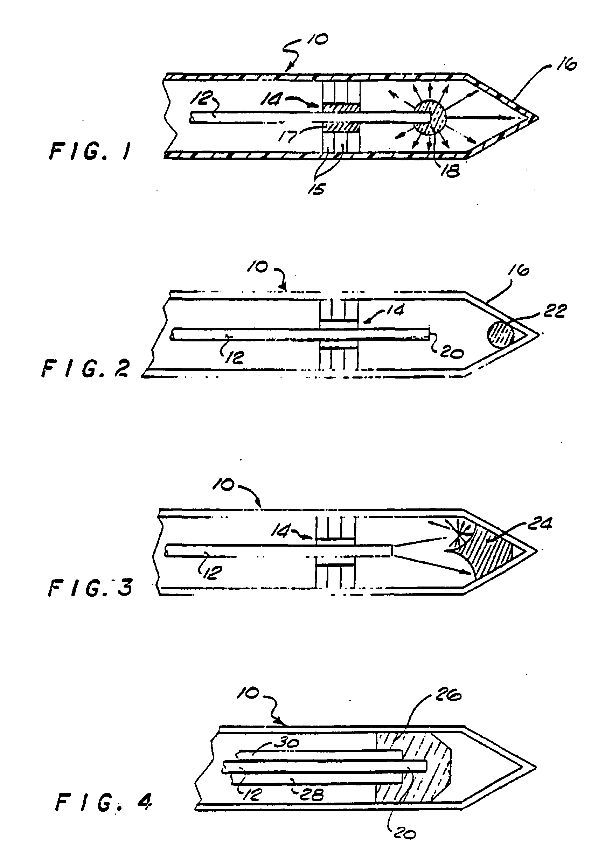

Referring initially to FIGURE 1, a preferred form of a laser lucent apparatus constructed according to the present invention is formed of a hollow, generally cylindrical, transparent needle 10, concentrically disposed about a commercially available optic fiber 12. The needle has a shaft and a tip and is preferably constructed of optical quality plastic, such as polycarbonate or polymethylmethacrylate (PMMA), so that the needle 10 may transmit laser energy at least through a substantial portion of the length of the shaft. As illustrated in FIGURE 1, energy may radiate outward from the fiber 12 and be transmitted through the tip and shaft of the needle, including a portion of the shaft located behind the tip of the fiber.

In a preferred embodiment, the outside diameter of the needle 10 may be as large as 3 millimeters. However, the size of the outside diameter will be determined by the desired usage of the present invention. The needle wall is thin relative to the needle diameter, e.g., 0.3 millimeters, to minimize the heating of the wall resulting from the transmission of energy from the fiber 12. Generally, a wall thickness of 0.1 to 1.0 millimeters is useful.

The fiber 12 is connected at its distal end to a laser source (not shown), and extends into a penetrating portion of the needle 10, i.e. a portion that is intended to penetrate into the tissue to be subjected to hyperthermia and/or photodynamic therapy. The fiber 12 is retained in position in the center of the needle 10 by retaining means or a spacer 14 which bridges the distance between the outside surface of the fiber 12 to the interior surface of the needle 10. Additional spacers may be incorporated and disposed along the length of the fiber 12 as required to retain the fiber 12 in position within the needle 10. In a preferred embodiment, the spacer 14 is porous to allow the flow of gas or liquid throughout the needle 10. In this particular embodiment, the spacer 14 has porous, flexible disks 15 of polytetrafluorethylene (Teflon) and an inner layer 17 of polycarbonate.

In use, the needle 10 and fiber 12 assembly is inserted within tissue as a means to help protect the optical fiber 12 from fracturing or shedding. Although not shown, the needle 10 may be open at the end proximal to the energy transmitting end of the fiber 12, as dictated by its usage. However, it is preferred that the proximal end of the needle be closed.

As illustrated in FIGURE 1, a closed end 16 of the needle 10 may be tapered or conically shaped. However, an infinite variety of shapes which are heat-formed made with inserts, epoxy-dipped or other suitable means may be used. The closed end 16 prevents any fragments of the fiber from exiting the needle 10, and introducing fiber fragments onto the tissue. It also prevents body fluids, such as blood, from entering the space between the fiber 12 and the needle 10, and thus interfering surrounding tissue. The distal end of the needle is open to provide for the connection of the fiber 12 to a laser source (not shown).

In tissues having moderate scattering, the laser lucent apparatus must deliver energy to the outside surface of the needle 10 with both an acceptably low and uniform energy density and a correct angle of incidence. To effect this, a diffusing tip 18 may be placed on the energy transmitting end 20 of the fiber 12. Such diffusing tips are usually formed of quartz and are commercially available, such as the 4400 Series spherical and cylindrical diffusers from LaserTherapeutics, Inc. The incorporation of a diffusing tip 18 within the interior of the needle 10 of the present invention results in an increase in the diffusion of the laser energy prior to its contact with the tissue. The increased area of the needle surface, as compared to the tip area, greatly lowers the power density at the tissue interface. This alleviates a problem of overheating at the tissue/tip interface present when a bare tip is used, while irradiating the same volume of tissue.

When the closed end 16 of the needle 10 is not in intimate contact with the tissue or fluid to be irradiated, an air void may be present in front of the closed end 16 of the needle 10. In air, the point of the closed end 16 is a light-focusing element which would produce a hot spot in front of the end of the needle. To counteract this effect, as illustrated in FIGURE 2, a scatterer 22 may be positioned adjacent to the closed end 16 of the needle 10. The scatterer 22 may take any of many forms, such as a spherical ball formed of plastic and loaded with refractive scattering formed of plastic and loaded with refractive scattering powder such as alumina, or may take other forms suitable for diffusing energy. The exact location of the scatterer 22 is not critical. However, the scatterer 22 acts to diffuse the energy transmitted from the energy transmitting end 20 of the fiber 12 and to prevent the overheating of the closed end 16 of the needle 10. A particular scatterer of the form shown in FIGURE 2 can be obtained by mixing 30% of alumina in epoxy and forming the material into balls of a suitable size, e.g., about 1.5 millimeters diameter.

FIGURE 3 illustrates the incorporation of an alternative form of the scatterer. In the embodiment illustrated in FIGURE 3, a reflector or mirror 24 diffuses the energy transmitted from the fiber 12. A concave annular mirror is shown, however, alternative reflector shapes will result in varied irradiation patterns. Moreover, it is not necessary that the mirror 24 be a total reflector, as a partial reflector incorporated with a diffusing scatterer in the mirror body will combine both effects and require less precise positioning within the needle 10.

FIGURE 4 illustrates the incorporation of a scatterer which is adapted to retain the energy transmitting end 20 of a bundle of fibers 12, 28 and 30 in position within the needle 10. A retainer scatterer 26 is preferably made of optical quality plastic, such as optical epoxy loaded with about 30% by weight of refractive scattering powder, such as sapphire powder and shaped such that the energy transmitting end 20 of the fibers may be matingly received within its core. As illustrated, the retainer scatterer 26 may be an abbreviated cylinder with bores extending partially through its central axis. The diameters of the bores are slightly larger than the diameter of the fibers 12 to hold the fibers 12 in a concentric position within the retainer scatterer 26. The outside surface of the scatterer 26 is constructed to abut the interior surface of the cylindrical portion of the needle 10 and its forward face is bevelled to somewhat radially distribute the laser light energy. As illustrated, the retainer spacer 26 may replace the spacer 14 as the means for retaining the fiber 12 in position within the needle 10.

The incorporation of a bundle of fibers, including a second fiber 28 and a third fiber 30 is made possible by the diameter of the needle 10. It should be understood that the number of optical fibers incorporated may be modified according to the desired usage. The energy transmitting ends of the fibers may also take several forms, such as flat or slanted, to control the angular spread of the transmitted energy.

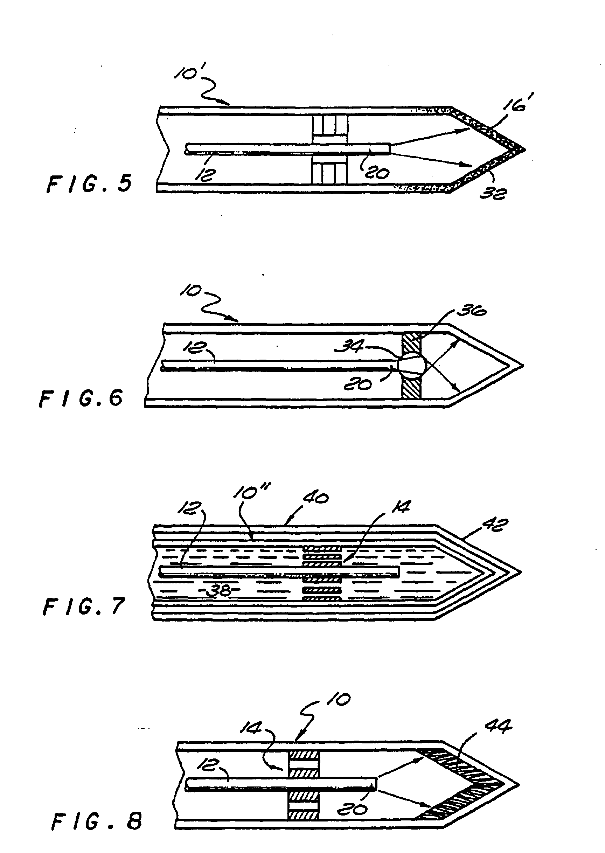

In the case of highly-scattering tissue, only uniform spreading of the energy is required (the angle of incidence becomes irrelevant). FIGURE 5 illustrates the incorporation of a flat-ended fiber 12 which is mounted a spaced distance from the closed end 16^{I} of the needle 10^{I}. The distance between the transmitting end of the fiber and the closed end of the needle determines the spread of the laser beam. By placing the energy transmitting end 20 of the fiber set back from the closed end 16 of the needle 10, the angular spread of the energy transmitted is increased. The incorporation of refractive scattering material 32, such as diamond powder, within the walls and the closed end of the needle 10 serves to diffuse the energy transmitted from the fiber. The scattering material 32 may be placed in the walls by admixture prior to forming the needle.

Referring to FIGURE 6, the incorporation of a lens positioned at the energy transmitting end 20 of the optical fiber 12 is illustrated. Divergence of energy transmitted from the fiber 12 may be induced by bringing the energy to a focus with a lens 34 positioned at the energy transmitting end 20 of the fiber 12. In the embodiment illustrated in FIGURE 6, a spherical lens is mounted'at the end of the fiber 12 and retained in position within the interior of the needle 10 by a lens support 36 formed of Teflon and connecting the exterior surface of the lens 34 to the interior wall of the needle 10. The lens 34 may be a spherical lens, a high-retractive-index negative lens e.g. of sapphire, or any other suitable lens capable of diverging the energy. As described above, the amount of illumination of the needle length may be altered by modifying the position of the lens 34 and the energy transmitting end 20 of the fiber 12 relative to the closed end 16 of the needle 10.

The use of laser technology to treat tumors requires matching of the heated volume to the size of the tumor. Consequently, a laser lucent apparatus for use in the treatment of tumors must be able to meet the requirements of a changeable irradiation wavelength, and thus a changeable penetration depth of the energy. FIGURE 7 illustrates a preferred embodiment of the present invention which utilizes fluorescent dye conversion to modify a laser pump wavelength. As illustrated in FIGURE 7, the optical fiber 12 is concentrically positioned within the needle 10^{II} by one or more porous spacers 14. For treating tumors, lasers in the blue to green range are preferred, such as argon ion lasers. A liquid suitable for fluorescent conversion, such as a fluorescent dye 38, is disposed within the interior of the needle 10^{II}. A fluorescent dye in the color range from orange to near-infra red is preferred, however any suitable dye may be used. Although not shown, circulating means may be added to the laser lucent apparatus to circulate the dye solution through the interior of the needle 10^{II} for cooling of the dye. An added benefit of having a cooled liquid in the needle 10^{II} is that it will provide more control over the steady-state thermal profile established in the surrounding tissue, both preventing the overheating of the needle-tissue interface and keeping the inner temperature gradient flat.

Fluorescent dyes are generally carcinogenic, thus requiring safety considerations to prevent the leaking of the dye 38 from the interior of the needle 10. To this end, a second needle 40 is illustrated in FIGURE 7. The second needle 40 is also constructed of a optical quality plastic with a configuration similar to that of the needle 10^{II}. However, the second needle 40 is configured to enclose the first needle 10^{II} within its interior.

The shape of the second needle 40 is essentially a hollow cylinder with a closed end 42. The diameter of the second needle 40 is sufficiently larger than the diameter of the needle 10^{II} to allow the second needle 40 to completely enclose the first needle 10^{II} within its interior while allowing an interstitial space between the exterior surface of the first needle 10^{II} (either by coaxial cantilever support (not shown) or by one or more spacers and the interior surface of the second needle 40. Thus, the second needle 40 provides a second barrier between the fluorescent dye 38 positioned within the interior of the first needle 10^{II} and the surrounding environment. Safety considerations suggest a monitoring of the interstitial space between the first needle 10^{II} and the second needle 40 for the appearance of leaking fluorescent dye.

In alternative embodiments for needles appropriate in treating tumors, an energy converter is incorporated, and more particularly, a solid-dye insert 44, positioned adjacent to the closed end of the needle 10, as illustrated in FIGURE 8, facing the energy transmitting end 20 of the fiber 12 which is supported by one or more spacers 14. The solid-dye insert 44 may have many shapes depending on the desired usage and the desired area of illumination of the needle 10. Such an insert may be formed of polymethylmethacrylate (PMMA) or polyvinyl chloride (PVC) containing 0.01 to 10% of dye, for example, PMMA containing 1% of Rhodamine 6G. An alternative embodiment of the dye insert 44 includes the addition of a coating of a dielectric reflector on the insert 44 to reflect inward-emitted radiation back into the tissue. Cooling of this embodiment of the present invention may be attained by the circulation of air within the interior of the needle 10 or the circulation of a liquid, such as saline.

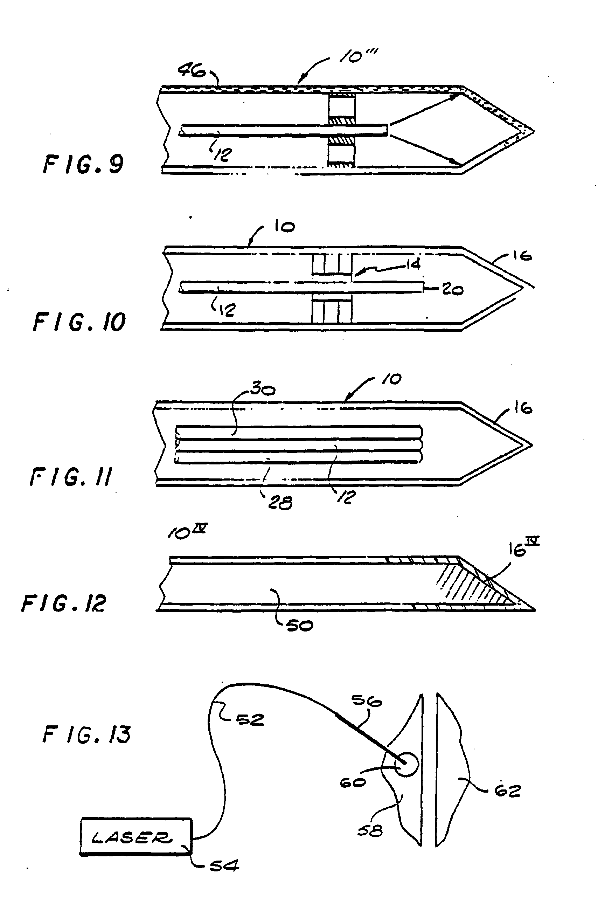

As illustrated in FIGURE 9, an energy converting material, such as a dye 46, e.g., Rhodamine 6G, may be embodied within the walls and closed end of the needle 10^{III} during the polymerization of the plastic. The energy converting dye 46 converts the wavelength of the energy transmitted from the fiber 12 to increase its suitability for treating tumors. This embodiment of the present invention may be cooled in the same manner as described in the paragraph above.

Referring to FIGURES 10 and 11, more generalized laser lucent needle structures are provided. The device of FIGURE 10 is similar to that of FIGURE 2, but omits the scatterer 22 of FIGURE 2 for those applications where control of the laser energy applied is at lower levels than with the device of Figure 2 or where a hot, undiffused tip is desired. The device of Figure 11 is similar to that of FIGURE 4 but omits the retaining scatterer of FIGURE 4; the forward faces of the individual fibers are bevelled.

In FIGURE 12, the needle 10^{IV} is open ended, at 16^{IV}. A solid stylus 50, e.g. of metal, sized to slidably close-fit the interior of the needle 10^{IV}, is disposed within the needle 10^{IV}, and facilitates insertion of the open ended needle 10^{IV}, preventing tissue or body fluid from entering the needle 10^{IV}. After insertion, the stylus 50 can be withdrawn and replaced by either one or more optical fibers or by a specialized instrument. For example, viewers or snippers or other instruments having a clinical purpose, such as for biopsies or for spectrographic tissue analysis, can be inserted and withdrawn through the needle. Such clinical procedures can be conducted before or after hyperthermia treatment of the tissue by either first placing laser optical fibers in the needle followed by replacement with the desired instrument, or vice-versa.

Referring to FIGURE 13, a method for treating BPH is shown schematically. An optical fiber 52 receiving light from a laser 54 is placed in a needle casing 56. The fiber-needle structure 52-56 can take any of the forms previously discussed herein with respect to rectum, guided visually by means of ultrasound, as known. The tissue in a region 60 surrounding the center of the lobe 58 is heated to a temperature of about 45-52°C for a period of time of from about one to about 30 minutes. This heating induces a slow necrosis of the central tissue volume of the gland, and dead cells are eventually metabolized, thus reducing the total mass of the gland. The procedure can be repeated for the other lobe 62.

By delivering the laser light through an optical fiber inserted with its tip in the targeted tissue, the region of elevated temperature is limited to exclude the capsule of the gland or any tissue external to the gland itself. Use of a needle encasing structure of any of the preceding FIGURES serves to make this procedure safe and effective with minimum side effects.

Although described for purposes of clarity with regard to specific preferred embodiments, the present invention is not limited to those embodiments, but rather is applicable broadly to all versions falling within the scope and spirit of the appended claims. For example, the needle may be equipped with surface thermocouples with wires carried through the interior. Additionally, fluoroscopic tracking may be facilitated by the addition of markers made of an appropriate material which are coated on the outside of the needle 10 distal to the radiation area. Asymmetric energy deposition may be achieved by adding mirror strips to the interior wall of the needle, where they may be cooled. Finally, the addition of a light return monitor to the laser lucent apparatus of the present invention may be incorporated to detect a sharp increase in energy from the fiber, which would indicate fiber breakage.

## Claims

1. Apparatus for the transmission of laser energy to tissue, comprising:
a hollow, laser light lucent needle having a shaft and a tip, and adapted to transmit laser energy at least along a substantial portion of the length of the shaft and the tip; and
at least one optical fiber disposed within the needle and extending into a penetrating portion of the needle, the fiber being adapted to transmit laser energy from its end adjacent said penetrating portion;
the needle having a configuration such that its interior surface is spaced from the exterior surface of the fiber.

2. The apparatus of claim 1 including a spacer formed to axially support the fiber within the needle.

3. The apparatus of claim 2 wherein the end of the penetrating portion of the needle is tapered.

4. The apparatus of claim 1 wherein the end of the penetrating portion of the needle is closed.

5. The apparatus of claim 4 wherein the energy transmitting end of the fiber is spaced from the closed end of the needle.

6. The apparatus of claim 1 wherein the end of the penetrating portion of the needle is open.

7. The apparatus of claim 6 including a solid stylus in said needle slidably close-fit to the interior surface of the needle.

8. The apparatus of claim 1 wherein the fiber includes a diffusing tip at its energy transmitting end, the tip being adapted to diffuse the energy transmitted from the fiber.

9. The apparatus of claim 1 further comprising a scatterer positioned adjacent the closed end of the needle, and adapted to diffuse the energy transmitted from the fiber.

10. The apparatus of claim 2 wherein the scatterer is spherical.

11. The apparatus of claim 9 wherein the scatterer is a reflector.

12. The apparatus of claim 2 wherein the spacer is a scatterer positioned adjacent to the closed end of the needle, and adapted to diffuse the energy transmitted from the fiber.

13. The apparatus of claim 4 wherein the closed end of the needle further includes refractive scattering material adapted to diffuse the energy transmitted from the fiber.

14. The apparatus of claim 4 wherein the needle further includes refractive scattering material positioned within the walls and closed end of the needle, the refractive material being adapted to diffuse the energy transmitted from the fiber.

15. The apparatus of claim 1 wherein the fiber includes a lens at its energy transmitting end, the lens being adapted to diffuse the energy transmitted from the fiber.

16. The apparatus of claim 15 wherein the lens is spherical.

17. The apparatus of claim 15 wherein the lens is a high-refractive-index negative lens.

18. The apparatus of claim 4 further comprising a liquid disposed within the needle.

19. The apparatus of claim 18 wherein the liquid is a fluorescent dye.

20. The apparatus of claim 18 further comprising a second hollow, transparent needle adapted to transmit laser energy;
the second needle being shaped such that first needle is receivable within its interior with the interior surface of the second needle spaced from the exterior surface of the first needle.

21. The apparatus of claim 4 further comprising an energy converter positioned adjacent the closed end of the needle, and adapted to convert the wavelength of the energy transmitted from the fiber.

22. The apparatus of claim 4 wherein the needle further includes wavelength-converting material positioned within the walls and closed end of the needle, the converting material being adapted to convert the wavelength of the energy transmitted from the fiber.

23. A method for treating benign prostate hypertrophy comprising inserting at least one optical fiber into a lobe of a prostate gland and directing laser light through said optical fiber into said lobe to heat tissue of said lobe to an elevated temperature for a time sufficient to induce necrosis of said tissue.

24. The method of claim 23 in which said optical fiber is contained in a laser light lucent needle.

25. The method of claim 24 in which said needle has a shaft and tip, and is adapted to transmit laser energy at least along a substantial portion of the length of the shaft and through the tip, said optical fiber being disposed within the needle and extending into a penetrating portion of the needle, the fiber being adapted to transmit laser energy from its end adjacent said penetrating portion, said needle having a configuration such that its interior surface is spaced from the exterior surface of the fiber.

26. The method of claim 23 in which said optical fiber is inserted into the approximate center of said lobe.

27. The method of claim 26 in which tissue adjacent said optical fiber is heated to about 45-52°C for a period of from about one to about 30 minutes.
